# EUROPEAN PATENT SPECIFICATION

(11) **EP 4 015 819 B1**
(45) Date of publication and mention of the grant of the patent: **22.05.2024**
(21) Application number: 21214333.3
(22) Date of filing: 14.12.2021
(51) Int. Cl.: F04B 19/00, F04B 23/02, F04B 53/02, F04B 53/16

(54) **MICROPUMP HAVING A SEALING RING**
MIKROPUMPE MIT DICHTRING
MICROPOMPE DOTÉE D'UNE BAGUE D'ÉTANCHÉITÉ

(30) Priority: 18.12.2020 GB 202020077
(43) Date of publication of application: 22.06.2022
(73) Proprietor: Merxin Ltd, King's Lynn, Norfolk PE30 5BY (GB)
(72) Inventor: STUART, ADAM, King s Lynn, PE31 1BS (GB)
(74) Representative: Basck Limited

(56) References cited:
- EP-A1- 1 942 967
- EP-A1- 2 700 692
- EP-A1- 3 378 894
- US-A- 4 892 232
- US-A1- 2012 325 204
- US-A1- 2018 086 901

## Description

### TECHNICAL FIELD

The present disclosure relates generally to micropumps for delivery of fluids; and more specifically, to sealing rings for the micropumps.

### BACKGROUND

Micro liquid dispensing systems have long been utilised as economical, and convenient devices for dispensing of diverse fluids. These systems are capable of dispensing small quantities of fluids within flow rates of micro/millilitres per minute. Such systems are of special interest as a robust fluid delivery method in a host of important applications such as controlled and accurate drug delivery, dispensing paint, chemicals delivery, and so forth. Furthermore, openings of the micro-pumps are sealed using sealing arrangements for efficient administration of flow of fluid therein.

However, a micropump as employed herein typically has very small components thereby making designing and construction of such a micropump complicated. Furthermore, deploying a sealing arrangement in a small component (namely, a sealing groove) makes construction of the micropump tedious and challenging. In an example, precise deployment of the sealing arrangement may necessitate cavity sorting of components in order to achieve the precision and match of the sealing arrangement in the sealing groove. Specifically, the sealing groove may have restricted and precise tolerances owing to small size thereof. Additionally, operation of the micropump under high pressure deforms the sealing arrangement and may thereby cause extrusion of the sealing arrangement out of the sealing groove. Moreover, slight deviation with respect to tolerances for accommodation of the sealing arrangement in the sealing groove further augments risks associated with extrusion of the sealing arrangement. Furthermore, high contact pressure in a micropump contributes to wearing of the sealing arrangement.

Additionally, the seal arrangement is subjected to high friction owing to pressurised operation thereof. Thus, the seal arrangement may undergo shredding resulting in a reduced life of such a sealing arrangement. Furthermore, filtering systems may need to be employed to prevent debris generated from the shredding of the seal arrangement from blocking the micropump and/or being dispensed from the micropump. Such filtering systems aggregate a manufacturing cost associated with the micropumps thereby making them less economical for large scale productions.

In US 7,284,484-B2, it is proposed to use a sealing material with a high gas permeation coefficient for nitrogen, a radial compression of <30%, and for this to fill the sealing groove to >90%. However, such filling of the sealing groove may lead to high tolerance sensitivity both on the sealing component and the groove, potentially requiring further in-process steps to achieve the required precision.

EP1942967 proposes a method and a device for metering a medicament, preferably a liquid. In order to achieve an improved metering accuracy, a first component manufactured in batches, such as a shaped seal, is combined with a second component, such as a delivery tube, of a matching group. This document does not disclose a groove having an axial size greater than the axial size of the seal so that the seal is operable to move axially inside the groove. Further, it does not disclose a seal made of a rubber composition comprising hydrogenated nitrile butadiene rubber and carbon black.

US20180086901 discloses an elastomer nanocomposite comprising an elastomer comprising a non-functionalized first elastomer and a functionalized second elastomer and a functionalized filler crosslinked with the elastomer.

Therefore, in light of the foregoing discussion, there exists a need to overcome the aforementioned drawbacks associated with prior sealing arrangements in micropumps.

### SUMMARY

The present disclosure seeks to provide a micropump with robust seals. The present disclosure seeks to provide a solution to an existing problem with micropumps; that of wear of a sealing ring that forms a sliding seal with a piston of the micropump. An aim of the present disclosure is to provide a solution that overcomes at least partially the problems encountered in prior art, and provides a rubber composition for manufacturing the sealing ring.

In one aspect, an embodiment of the present disclosure provides a micropump for delivery of a fluid, comprising
- a body comprising a metering chamber and a groove;
- a capillary for delivering the fluid into the metering chamber;
- the metering chamber for receiving the fluid, wherein the fluid is delivered through a nozzle attached downstream of the metering chamber;
- a piston operable to move axially inside the metering chamber and having a piston face at a first end of the piston, wherein an axial movement of the piston face along a path between a second end and a first end of the metering chamber delivers the fluid through the nozzle;
- the groove radially surrounding the piston and having a common axis with the piston, wherein the groove comprises a sealing ring, wherein the axial size of the groove is greater than the axial size of the sealing ring so that the sealing ring is operable to move axially inside the groove based on the axial movement of the piston and wherein the sealing ring is in contact with the piston and the body;
wherein the sealing ring is manufactured using a rubber composition comprising
- hydrogenated nitrile butadiene rubber (HNBR), and
- carbon black.

Embodiments of the present disclosure substantially eliminate or at least partially address the aforementioned problems in the prior art, and enables cost-efficient large-scale production of micropumps by improving a quality of sealing rings employed in the micropumps.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:
FIG. 1 is a cross section of a micropump for delivery of a fluid, in accordance with an embodiment of the present disclosure, but the capillary has been omitted to aid in seeing other aspects of the mechanism;
FIGs. 2 and 3 are cross sections of a micropump in operation, for delivery of a fluid, in accordance with different exemplary implementations of the present disclosure;
FIG. 4A is an expanded representation of a photograph of a sealing ring taken from a micropump of the invention after 100 actuations; and
FIG. 4B is an expanded representation of a photograph of a sealing ring taken from a micropump of the prior art after 100 actuations.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

The present disclosure provides a micropump for delivery of a fluid comprising a sealing ring. The sealing ring provides a sliding seal with the piston. The sealing ring is typically an 'O' ring. In this regard, a rubber composition for the sealing ring in the micropump provides efficient sealing in the micropump that enables regulated delivery of the fluid in accurate manner. Specifically, the sealing ring employs hydrogenated nitrile butadiene rubber that maintains performance in terms of providing a sliding seal against high pressure fluid that retains the fluid, while keeping the friction between the seal and the piston at an acceptable level and providing stability against wear. Furthermore, the increased Shore A hardness of the sealing ring alleviates extrusion of the sealing rings from the cylindrical groove. Moreover, enhancing the Shore A hardness of the sealing ring allows for larger gaps between components of the micropump thereby relaxing tolerance for manufacturing of the components of the micropump. Additionally, the sealing ring is allowed to move inside the cylindrical groove thereby preventing high tolerance sensitivity, potential extrusion of the sealing ring and wear of the sealing ring owing to high friction. Specifically, reducing tolerance for manufacturing of the components enables easier control of components of the micropump, thereby making the manufacturing process economic and less cumbersome. Furthermore, the sealing ring as discussed in the present invention withstands extrusion at high pressure operations of the micropump. Moreover, the sealing ring has enough wear resistance to prevent degradation thereof during conditions of higher contact pressure and high friction. Furthermore, significant reduction in wear of the sealing ring further allows for removal of filtering component or some parts of the filtering component in the micropump thereby reducing a cost of manufacture of the micropump. Such reduction in cost of micropumps owing to longevity of the sealing ring and reduction in manufacturing cost of the micropump aggregates to make the micropump economical thereby mobilizing large scale consumption of the micropump.

The present disclosure provides a micropump for delivery of a fluid. The micropump comprises a body comprising a metering chamber for receiving the fluid and a groove, a capillary for delivering the fluid into the metering chamber, a piston operable to move axially inside the metering chamber, and a nozzle attached downstream of the metering chamber for delivery of fluid therethrough. Typically, an axial movement of a piston face at a first end of the piston along a path between a second end and a first end of the metering chamber delivers the fluid through the nozzle. The groove radially surrounds the piston and has a common axis with the piston. Moreover, the groove comprises a sealing ring, wherein the axial size of the groove is greater than the axial size of the sealing ring so that the sealing ring is operable to move axially inside the groove based on the axial movement of the piston and the sealing ring is in contact with the piston and the body. Furthermore, the sealing ring is manufactured using a rubber composition comprising hydrogenated nitrile butadiene rubber (HNBR), and carbon black.

Throughout the present disclosure, the term *"micropump"* refers to micro liquid dispensing systems employed to dispense a fluid in small quantities with increased accuracy. Furthermore, the micropump can control and manipulate small volumes of fluid (generally in micrometric volumes). Typically, a pressure force is exerted to dispense the fluid from the micropump through a narrow outlet (such as a nozzle). Furthermore, the narrow outlet allows the fluid to be delivered with a higher pressure.

Materials used for manufacturing the components of the micropump include, but are not limited to, metals, alloys, non-metals (such as semi hardened polyvinyl materials, polymeric materials, glass) or a combination thereof. In an embodiment, at least one of the components of the micropump, i.e. the body, including the metering chamber, the capillary and the piston, is manufactured using polyoxymethylene, commonly referred to as Acetal.

Throughout the present disclosure, the term *"fluid"* refers to a substance that undergoes a deformation in shape and/or volume when subjected to an external force. The fluid is typically a liquid. It will be appreciated that such substances are required to be delivered by the micropump in small quantities for delivery of accurate volume and at precise locations of delivery. Examples of the fluid include, but are not limited to, inhalation aerosol drug formulations. One aspect of inhalation drug formulations is the need to provide a spray with a droplet size small enough to penetrate to the lungs. In order to produce such a spray by forcing fluid through nozzles, it is necessary to force the fluid at high pressure, e.g. 10 to 59MPa.

Optionally, the fluid comprises a pharmaceutical compound. Notably, the pharmaceutical compound may be dissolved in a suitable solvent. In this context, a suitable solvent could be water or other pharmaceutically acceptable low volatility materials. Specifically, the fluid delivered by the micropump is a substance used as a medication, i.e. the fluid is chemical compound that has a physiological effect on the patient when administered. In an example, the pharmaceutical compound may be tiotropium bromide.

The micropump comprises a body comprising a metering chamber and a groove. The body refers to a main hollow cylindrical structure that retains the components of the micropump and is designed to withstand high pressure and minimize leakage. It may include additional components such as a nozzle holder and/or a nozzle piece, and sealing elements between such components. It may also have screw-on end caps at each end that retain the components of the micropump within the body. Moreover, the body comprises at least one aperture (namely, the nozzle) thereon required for the delivery of the fluid.

The micropump comprises the metering chamber for receiving the fluid. The metering chamber refers to a hollow volume provided in the body of the micropump to hold the fluid prior to the delivery thereof. Furthermore, the metering chamber receives a metered volume of fluid therein. In an example, the metered volume of fluid is predefined and may be altered based on requirement of an amount of the fluid.

Furthermore, the fluid is delivered through a nozzle attached downstream of the metering chamber. The nozzle refers to the outlet for the fluid on the body of the micropump. Notably, the nozzle enables an efficient delivery of the fluid at an intended location of delivery. Additionally, downstream refers to a direction of flow of fluid, specifically, from the metering chamber towards the nozzle. It will be appreciated the nozzle is implemented based on a manner of delivery of fluid required (such as a jet stream, a spray, a sprinkle, a diffusion) based on the application of the micropump. In an example, the nozzle may be fabricated in a cylindrical shape, and may include a plurality of miniscule openings. Beneficially, the plurality of miniscule openings allow the fluid to be passed through to deliver the fluid in sprayed manner. In another example, the nozzle may include shapes such a conical, semi-spherical, and so forth.

The micropump for delivery of a fluid comprises a capillary for delivering the fluid into the metering chamber. The capillary refers to a hollow cylindrical tube arranged inside the micropump to carry the fluid therein. Typically, the diameter of the capillary is smaller than the length of the capillary. Beneficially, a small diameter of the capillary assists in delivery of an accurately metered quantity of the fluid. It will be appreciated that the capillary connects a receptacle containing the fluid therein to the metering chamber, wherein a metered volume of the fluid is delivered to the metering chamber.

In an embodiment, the receptacle containing the fluid is arranged outside the micropump, wherein the receptacle is detachably attached to the micropump. Specifically, the body of the micropump comprises an aperture arranged thereon. Consequently, the receptacle is attached to the aperture and subsequently, the fluid to be delivered is drawn through the aperture into the capillary and delivered therethrough to the metering chamber. It will be appreciated that, in such an instance, the receptacle is not an integral part of the micropump and is only attached to the micropump to draw the metered volume of the fluid therefrom and the receptacle may be detached thereafter.

In another embodiment, the receptacle containing the fluid is arranged inside the micropump. Specifically, the body of the micropump comprises the receptacle, and the capillary is arranged as a channel from the receptacle to the metering chamber.

The micropump for delivery of a fluid comprises a piston operable to move axially inside the metering chamber and having a piston face at a first end of the piston. The piston refers to a cylinder, or a disc attached to a connecting rod, arranged inside the metering chamber of the micropump to effect delivery of the fluid through the nozzle by developing a pressure in the metering chamber. Specifically, the axial movement of the piston inside the metering chamber pushes the fluid through the metering chamber towards the nozzle. Notably, in an implementation, the piston face at the first end of the piston is in contact with the fluid in the metering chamber. Furthermore, the piston is arranged inside the metering chamber such that there is significantly low clearance between outer walls of the piston and inner walls of the metering chamber. It will be appreciated that the piston may further comprise a second end, wherein a pressure force is applied at the second end. Consequently, the pressure force is transferred to the piston face at the first end of the piston to effect delivery of the fluid.

Furthermore, an axial movement of the piston face along a path between a second end and a first end of the metering chamber delivers the fluid through the nozzle. Notably, the metering chamber comprises the first end and the second end, wherein the nozzle is attached proximate to the first end of the metering chamber. In operation, when the fluid is received by the metering chamber, the first end of the piston (comprising the piston face) is proximate to the second end of the metering chamber. Subsequently, the piston is moved axially inside the metering chamber from the second end of the metering chamber to the first end. Such movement of the piston from the second end of the metering chamber to the first end pushes and delivers the fluid through the nozzle proximate to the first end. Such delivery of the fluid through the nozzle may constitute a metered dose of product, for example when the micropump is used as part of drug delivery device, such as an inhaler. It will be appreciated that the metered volume delivered corresponds to the volume of the metering chamber swept by the piston in its stroke, which may be calculated from the cross-sectional area of the piston and the stroke length. The cross-sectional area may be about 2 mm², and the stroke about 8 mm, resulting in a metered volume of about 16µl. Accordingly, the micropump may be constructed to deliver a metered dose in the range 3 to 25 µl.

Subsequently, post-delivery of the fluid through the nozzle, the piston is pulled towards starting position thereof (namely, the position proximate to the second end of the metering chamber) for subsequent operation of the micropump.

Optionally, post-delivery of the fluid through the nozzle, when the piston is pulled towards the second end of the metering chamber, the piston may generate a suction force inside the metering chamber, and causes the fluid to travel from the receptacle through the capillary to the metering chamber.

Optionally, the capillary is implemented as a capillary bore through the piston. As mentioned previously, the micropump comprises the capillary for delivering fluid into the metering chamber. In such implementation, the capillary bore is arranged as a longitudinal cavity through the second end of the piston to the first end of the piston such that the fluid is delivered therethrough into the metering chamber. The capillary bore through the piston is coaxial to the piston, and runs along the length of the piston. Furthermore, the capillary bore connects to the receptacle containing the fluid to the metering chamber.

Optionally, the capillary comprises a valve arrangement to restrict backward flow of the fluid from the metering chamber. Specifically, the valve arrangement blocks the backward flow of the fluid from the metering chamber. More specifically, the valve arrangement provides a one-way path of travel for the fluid inside the capillary. In an example, the valve arrangement valves such as ball valve, gate valve, plug valve and so forth. Furthermore, size of the valve depends on the diameter of the capillary. Preferably, the diameter of the valve is greater than that of the capillary to provide the restriction when in operation. Furthermore, materials used for manufacturing the valve arrangement include, but are not limited to, metals, alloys, non-metals (such as semi hardened polyvinyl materials, polymeric materials, glass) or a combination thereof. In an embodiment, the valve arrangement is manufactured using polyoxymethylene, commonly referred to as Acetal.

Optionally, the delivery of fluid through the nozzle is driven by a force on the piston that causes a peak pressure to develop in the metering chamber in the range 10 to 59 mega Pascal (MPa). Optionally, the peak pressure has range 10 to 59 MPa. In an example, the pressure may be at least 10, 11, 12, 13, 14, 15, 16, 17, 18, 19, 20, 21, 22, 23, 24, 25, 30, 35, 40, or 45, and up to 24, 25, 26, 27, 28, 29, 30, 35, 40, 45, 50, 55 or 59 MPa of pressure. In an example, the effect of the applied force may be equal to 28 MPa. In order to create a peak pressure inside the capillary, a force is applied on the piston to deliver the fluid through the nozzle. In an example, the force may be applied at the second end of the piston. Typically, the force may be applied to a receptacle for fluid to which the piston is rigidly attached.

The micropump for delivery of a fluid comprises the groove radially surrounding the piston and having a common axis with the piston. Throughout the present disclosure, the term *"groove"* refers to a recess or a cavity provided in the micropump. Furthermore, the groove is arranged as a hollow space to make room for components to be adjusted therein. Specifically, a groove is arranged to accommodate one or more smaller components therein. Moreover, the groove is arranged to radially surround the piston such that the groove and the piston comprise a common axis. Specifically, the groove is arranged longitudinally surrounding the piston.

Furthermore, the groove comprises a sealing ring operable to move inside the groove based on the axial movement of the piston and wherein the sealing ring is in contact with the piston and the body. Furthermore, the groove radially surrounds the piston and coaxial with the piston and the sealing ring is arranged in the groove such that the piston passes through the sealing ring. Consequently, the sealing ring radially surrounds the piston and functions as a seal around the piston and prevents flow of the fluid past the groove. Additionally, a diameter of the groove is structured to accommodate a diameter of the sealing ring. The sealing ring refers to a circular ring or a circular tube that when in operation, undergoes deformation to form a secure seal between two surfaces that are in contact with the sealing ring. Furthermore, the sealing ring is dimensionally deformable, and also provides friction between two surfaces that are in contact with the sealing ring. In an example, a length of the groove is affixed and comprises one-tenth or one-twelfth of the length of the metering chamber. Furthermore, the groove is positioned proximate to the second end of the metering chamber inside the body. Additionally, a diameter of the groove is greater than the diameter of the metering chamber. In another example, a height of the sealing ring is equal to the half or one-third of the length of the groove. Furthermore, the sealing ring is operable to move inside the groove, and contact with the body and piston to provide a seal therein. Moreover, the frictional force between the two surfaces that are in contact with the sealing ring (the body and the piston), deforms the sealing ring and thereby creates a seal therein.

Optionally, the groove is cylindrical. As mentioned previously, the groove radially surrounds the piston. Therefore, a cylindrical groove around the piston forms a cylindrical cavity surrounding the piston. It will be appreciated that the diameter of the cylindrical groove is greater than the diameter of the piston. Furthermore, an outer diameter of the sealing ring may be equal to or less than the diameter of the cylindrical groove.

Optionally, the sealing ring in groove provides at least one of a radial seal, an axial seal with the piston. Furthermore, the sealing ring is operable to move along the groove to form a seal between the body and the piston. Specifically, the sealing ring provides a seal operable to seal radially to restrict a movement of the fluid along the radius of the micropump. More specifically, the sealing ring restricts a movement of the fluid radially inside the groove. It is to be understood that a radial movement relates to an outward movement away from a radius of a circle. In another example, the sealing ring provides a seal operable to seal axially to prevent an axial movement of the fluid past the piston. It will be appreciated that axial movement herein relates to a movement along a centre of a cylindrical structure i.e. the piston. In an instance, when the piston is drawn out of the metering chamber, the sealing ring is dragged against an axial face of the body forming an axial seal in addition to the radial seal. Particularly, when the piston is driven into the metering chamber, the pressure inside the metering chamber to rapidly increase and the axial seal between the sealing ring and the axial face of the piston to be increased. Furthermore, the high pressure inside the metering chamber causes the sealing ring to deform and mould against the same axial face of the body.

Optionally, the body includes two parts that are assembled to define the groove and retain the sealing ring. Moreover, the dimensions of the groove is based on the sealing ring that is to be accommodated therein. Thus, the volume of the sealing ring may be in the range 50 to 80% of the volume of the groove. Additionally, the body also accommodates the piston and the capillary therein. Therefore, during manufacturing process, internal dimensions of the body are based on dimensions of the seal, and the piston. In an instance, a diameter of the piston determines the diameter of the groove as well as the diameter of the metering chamber. Furthermore, the groove provides enough room for the sealing ring to roll axially inside the body.

The sealing ring is manufactured using a rubber composition, the rubber composition comprising hydrogenated nitrile butadiene rubber (HNBR), and carbon black. Furthermore, elastic rubber compositions are employed for manufacturing the sealing rings, such that deformations can be sustained. Beneficially, the hydrogenated nitrile butadiene rubber (HNBR) material does not extrude due to higher stiffness, and provides a resilient sealing thereto. Moreover, carbon black refers to a material produced by the incomplete combustion of heavy petroleum products. Notably, carbon black is a form of para-crystalline carbon that has a high surface-area-to-volume ratio. Additionally, the carbon black provides stiffness rigidity to the sealing ring, to regain original dimensions after deformations. Additionally, the hydrogenated nitrile butadiene rubber (HNBR) exhibits resilient property to actuations, loads, pressure, and so forth. Furthermore, the hydrogenated nitrile butadiene rubber (HNBR), used herein is obtained by hydrogenation of nitrile rubber, a synthetic rubber copolymer of acrylonitrile (ACN) and butadiene. Specifically, hydrogenated nitrile butadiene rubber is a derivative of nitrile rubber hydrogenated in a solution containing precious metal catalysts.

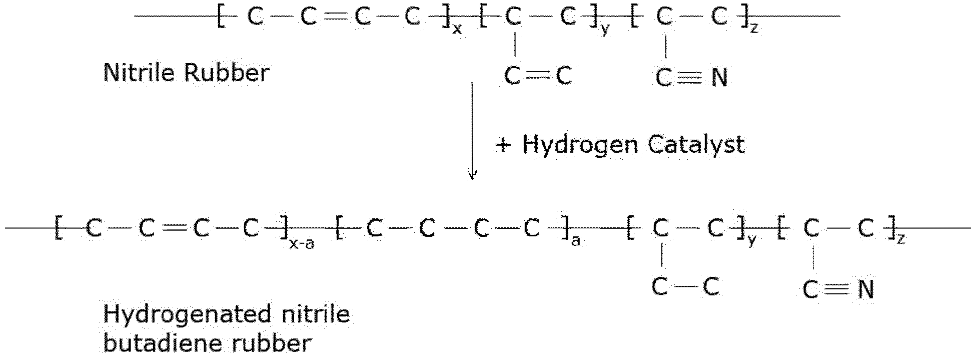

Optionally, the concentration of the carbon black in the rubber composition is in a range of 40 to 60 parts per hundred parts of rubber (PHR) with respect to hydrogenated nitrile butadiene rubber (HNBR). It will be appreciated that the PHR (parts per hundred parts of rubber by weight) as used in the disclosure is the one used in the rubber industry quantitative indication mix recipes. The dosage of the parts by weight of the individual substances is based on 100 parts by weight of the total mass of hydrogenated nitrile butadiene rubber (HNBR). Specifically, the carbon black in the rubber composition is added to provide structural rigidity to the rubber composition. Beneficially, carbon black improves resistance to wear of the sealing ring. Optionally, the concentration of the carbon black in the rubber composition is in a range of 40 to 60 parts per hundred parts of rubber (PHR). In an example, the range may be from 40, 41, 42, 43, 44, 45, 50, 55, or 59 up to 41, 42, 43, 44, 45, 50, 55, or 60 parts per hundred parts of rubber (PHR). More optionally, concentration of the carbon black in the rubber composition is 50 parts per hundred parts of rubber (PHR) with respect to hydrogenated nitrile butadiene rubber (HNBR).

Optionally, the sealing ring is manufactured using a rubber composition comprising at least one metal oxide selected from zinc oxide, magnesium oxide. In order to enhance the toughness and rigidity of the sealing ring metal oxide dopants such as zinc oxide, magnesium oxide are used. Specifically, addition of metal oxides to the rubber composition provides rigidity at a molecular level. Furthermore, such hybridization enhances structural rigidity of the rubber composition.

Optionally, the concentration of the zinc oxide in the rubber composition is in a range of 1 to 3 parts per hundred parts of rubber (PHR) with respect to hydrogenated nitrile butadiene rubber (HNBR). More optionally, the concentration of the zinc oxide in the rubber composition is 2 parts per hundred parts of rubber (PHR). In an example, the concentration range of zinc oxide may be from 1.0, 1.1, 1.2, 1.3, 1.4, 1.5, 2.0, or 2.5 up to 1.5, 1.6, 1.7, 1.8, 1.9, 2.0, 2.5, or 3 parts per hundred parts of rubber (PHR).

Optionally, the concentration of the magnesium oxide in the rubber composition is in a range of 1 to 3 parts per hundred parts of rubber (PHR) with respect to hydrogenated nitrile butadiene rubber (HNBR). As mentioned above, the disclosed rubber composition comprises the magnesium oxide in a range of 1 to 3 parts per hundred parts of rubber (PHR) with respect to hydrogenated nitrile butadiene rubber (HNBR), to provide resistance wear and tear. More optionally, the concentration of the magnesium oxide in the rubber composition is 2 parts per hundred parts of rubber (PHR). In an example, the concentration range of magnesium oxide may be from 1.0, 1.1, 1.2, 1.3, 1.4, 1.5, 2.0, or 2.5 up to 1.5, 1.6, 1.7, 1.8, 1.9, 2.0, 2.5, or 3 parts per hundred parts of rubber (PHR).

In an embodiment, the rubber composition is manufactured using a rubber composition comprising a peroxide crosslinking agent. It will be appreciated that the crosslinking agents refers to agents that link one chain of polymers to the other by means of covalent bonds or ionic bonds. Furthermore, crosslinking agents mechanically strengthen the rubber composition to enhance wear, actuations, and the like. Specifically, the peroxide crosslinking agent enhances bonding between the interlinking polymer chains. More specifically, peroxides when used as cross-linking agents form free-radicals that aid in cross-linking of chains of polymers in rubber.

Optionally, the peroxide cross linking agent is bis(t-butylperoxy isopropyl) benzene (BIPB), and the concentration of the cross linking agent in the rubber composition is in a range of 2 to 4 parts per hundred parts of rubber (PHR) with respect to hydrogenated nitrile butadiene rubber (HNBR). Notably, the chemical formula for bis(t-butylperoxy isopropyl) benzene is C₆H₄(C(CH₃)₂OOC(CH₃)₃]₂. The peroxide cross linking agent is often supplied with a carrier such that the actual content of this substance in the material supplied is diluted to e.g. 40% w/w. It will be appreciated that the concentration of the bis(t-butylperoxy isopropyl) benzene (BIPB) with respect to hydrogenated nitrile butadiene rubber (HNBR) depends on requirement of the toughness of the rubber composition. More optionally, the concentration of the cross linking agent in the rubber composition is 2.8 parts per hundred parts of rubber (PHR). In an example, the concentration range of cross linking agent may be from 2.0, 2.1, 2.2, 2.3, 2.4, 2.5, 3.0, or 3.5 up to 2.5, 2.6, 2.7, 2.8, 2.9, 3.0, 3.5, or 4 parts per hundred parts of rubber (PHR).

In another embodiment, the rubber composition is manufactured using a rubber composition comprising a sulphur curing agent. Specifically, the manufacturing process of the rubber composition comprises heating the rubber composition with the sulphur curing agent. Beneficially, rubber compositions cured using the sulphur curing agents have cross-links between sections of polymer chains, thereby significantly enhancing durability and rigidity of the rubber composition. Optionally, the sulphur curing agent is selected from a group consisting of, but not limited to, thiurams, sulphonamides and dithiocarbamates. Examples of thiuram compounds include, but are not limited to, Tetramethylthiuramdisulphide (TMTD), Tetra-benzylthiuramdisulphide (TBzTD) and Dipenta-methylene-thiuram tetrasulphide (DPTT).

Optionally, the rubber composition of the sealing ring is manufactured using a rubber composition comprising at least one of: anti-ager dialkylated diphenylamine (DDA), anti-ager zinc di(benzimidazole-2-yl)disulphide (MBZ), cross-linking agent Triallyl isocyanurate (TAIC). It will be appreciated that the anti-ager refers to chemical compounds, when added to a composition, act as a reagent to enhance the life span of the overall composition or substance. Furthermore, anti-agers used herein such as dialkylated diphenylamine (DDA), and zinc di(benzimidazole-2-yl)disulphide (MBZ) prolong life of the rubber composition and provide protection against decay or failure, when subjected to load fluctuations for a prolonged spans of time. Beneficially, in this regard, the sealing ring is cured with the cross linking agents to provide additional resistance to the sealing ring from wear owing to operation of the sealing ring under pressurized conditions. It will be appreciated that friction under pressurized conditions yields debris of sealing ring thereby blocking the nozzle of the micropump. Beneficially, filling the sealing ring with carbon black and further treating thereto with bis(t-butylperoxy isopropyl)benzene (BIPB) and Triallyl isocyanurate (TAIC) prevents formation of such debris of the sealing ring thereby preventing inaccurate delivery of fluids owing to blockage of the nozzle.

Optionally, the anti-ager dialkylated diphenylamine (DDA) in the rubber composition is in a range of 0.5 to 1.5 parts per hundred parts of rubber (PHR) with respect to hydrogenated nitrile butadiene rubber (HNBR). It will be appreciated that addition of the anti-ager dialkylated diphenylamine (DDA) enhances a durability of the rubber composition. In an example, for intermediate durability, the anti-ager dialkylated diphenylamine (DDA) is added in a concentration of 0.5 parts per hundred parts of rubber (PHR) with respect to hydrogenated nitrile butadiene rubber (HNBR). More optionally, the concentration of the anti-ager dialkylated diphenylamine (DDA) in the rubber composition is 0.4 parts per hundred parts of rubber (PHR). In an example, the concentration range of anti-ager dialkylated diphenylamine (DDA) may be from 0.5, 0.6, 0.7, 0.8, 0.9, 1.0, 1.1, 1.2, 1.3, 1.4 up to 0.6, 0.7, 0.8, 0.9, 1.0, 1.1, 1.2, 1.3, 1.4, 1.5 parts per hundred parts of rubber (PHR). Beneficially, anti-ager dialkylated diphenylamine (DDA) provides the rubber composition resistance to thermal oxidation, high static and dynamic stresses, and ozone aging.

Optionally, the concentration of the anti-ager zinc di(benzimidazole-2-yl)disulphide (MBZ) in the rubber composition is in a range of 0.2 to 0.6 parts per hundred parts of rubber (PHR) with respect to hydrogenated nitrile butadiene rubber (HNBR). Notably, the chemical formula of zinc di(benzimidazole-2-yl)disulphide (MBZ) is C₁₄H₁₀N₄S₂Zn. As mentioned earlier in the present disclosure, the anti-ager zinc di(benzimidazole-2-yl)disulphide (MBZ) is added in the rubber composition to enhance the durability of the sealing ring. Furthermore, based on applications and usage, range of the concentration of the anti-ager zinc di(benzimidazole-2-yl)disulphide (MBZ) in the rubber composition is obtained. More optionally, the concentration of the anti-ager zinc di(benzimidazole-2-yl)disulphide (MBZ) in the rubber composition is 0.4 parts per hundred parts of rubber (PHR). In an example, the concentration range of zinc di(benzimidazole-2-yl)disulphide (MBZ) may be from 0.2, 0.3, 0.4, or 0.5 up to 0.3, 0.4, 0.5, or 0.6 parts per hundred parts of rubber (PHR).

Optionally, the concentration of the cross-linking agent Triallyl isocyanurate (TAIC) in the rubber composition is in a range of 0.5 to 2.5 parts per hundred parts of rubber (PHR) with respect to hydrogenated nitrile butadiene rubber (HNBR). Notably, the chemical formula of Triallyl isocyanurate (TAIC) is C₁₂H₁₅N₃O₃. Furthermore, presence the cross-linking agent Triallyl isocyanurate (TAIC) in the rubber composition enhances the toughness and rigidity, to restrict a deformation of the sealing ring when subjected to extrusion. Moreover, the cross-linking agent Triallyl isocyanurate (TAIC) in the rubber composition increases a intermolecular bonding of the sealing ring, thereby withstands under certain temperature and pressure. More optionally, the concentration of the cross-linking agent Triallyl isocyanurate (TAIC) in the rubber composition is 1.5 parts per hundred parts of rubber (PHR). In an example, the concentration range of cross-linking agent Triallyl isocyanurate (TAIC) may be from 0.5, 0.6, 0.7, 0.8, 0.9, 1.0, 1.5, or 2.0 up to 1.0, 1.1, 1.2, 1.3, 1.4, 1.5, 2.0, 2.5 parts per hundred parts of rubber (PHR).

Optionally, the micropump for delivery of a fluid further comprises a gasket seal, whereby opposing surfaces of the gasket seal form face seals with the body and the nozzle, and whereby the gasket is manufactured using a rubber composition the same as that used for the sealing ring. The term *"gasket seal"* used herein refers a ring or a tube, when in operation, undergoes deformation to provide a seal between the body and the nozzle. Furthermore, the opposing surfaces of the gasket seal form face seals with the body and the nozzle to restrict the flow of the fluid therethrough. In an example, the gasket is manufactured using hydrogenated nitrile butadiene rubber (HNBR), and the carbon black. In another example, the gasket is manufactured using polyoxymethylene, commonly referred to as Acetal.

Optionally, the micropump further comprises a filter arranged between the metering chamber and the nozzle. Specifically, the filter is implemented prior to the nozzle to restrict any impurities in the fluid to be delivered with the fluid. The filter may further restrict any debris generated due to extrusion of the sealing ring. It will be appreciated that the filter is detachable from the micropump for cleaning and restoration purposes.

Optionally, the rubber composition has a Shore A hardness in a range of 70 to 90. The term *"Shore A hardness"* used herein refers to a measure of resistance of a material to penetration of a spring loaded needle-like indenter. The measure is indicative of mechanical properties of a rubber material defining toughness, rigidity, resistance to fatigue, and the like, when subjected to shear, stress, strain, and the like. Traditionally, the Shore A hardness chart defines hardness property of a wide range of rubber materials, having significant values assigned based tests and trials thereto. In an example, Shore A hardness of rubber band is Shore A 20, pencil eraser is Shore A 40, assigned in the class of soft and medium soft rubber materials. In another example, the Shore A hardness of tyre tread is Shore A 70, shoe heel is Shore A 80, assigned in the class of medium hard and hard rubber materials. In yet another example, the Shore A hardness of cart wheels rubber comes under extra hard rubber, and in range of Shore A 90-100. Specifically, the Shore A hardness of the rubber composition used herein comes in range of extra hard rubber materials. In yet another example the range of Shore A hardness may be in a range of 70, 71, 72, 73, 74, 75, 76, 77, 78 or 79, up to 91, 92, 93, 94, 95, 96, 97, 98, 99 or 100 Shore A hardness.

### DETAILED DESCRIPTION OF THE DRAWINGS

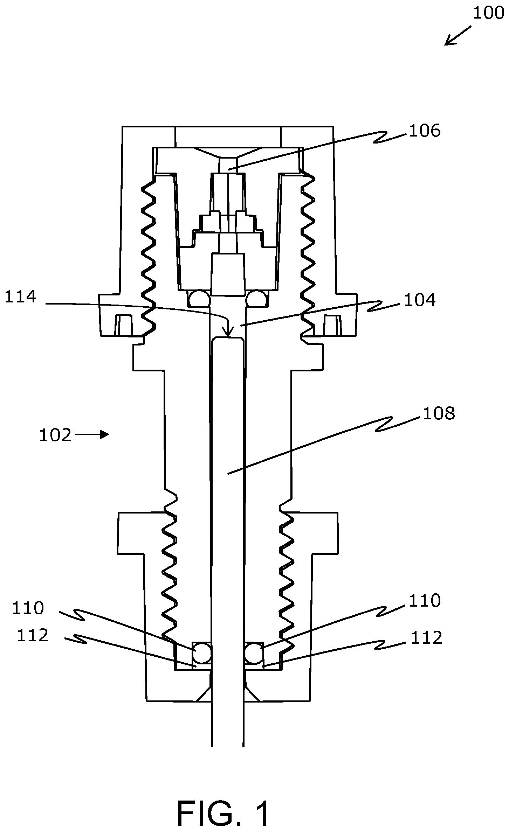

Referring to FIG. 1, there is shown cross section of a micropump **100** for delivery of a fluid, in accordance with an embodiment of the present disclosure. Notably, the capillary has been omitted to aid in seeing other aspects of the mechanism. As shown, the micropump **100** for delivery of the fluid comprises a body **102.** The micropump **100** comprises a capillary (not shown) for delivering the fluid into the metering chamber **104.** Notably, the capillary has been omitted to aid in seeing other aspects of the mechanism. Moreover, the body **102** comprises a metering chamber **104** for receiving the fluid. Specifically, the fluid is delivered through a nozzle **106** attached downstream of the metering chamber **104.** The micropump **100** further comprises a piston **108** operable to move axially inside the metering chamber **104** and having a piston face **114** at a first end of the piston **108.** Specifically, an axial movement of the piston face along a path between a second end and a first end of the metering chamber **104** delivers the fluid through the nozzle **106.** The body **102** of the micropump **100** further comprises a groove **112** which radially surrounds the piston **108** and has a common axis with the piston **108.** The groove **112** houses a sealing ring **110,** which is an 'O' ring. The sealing ring **110** is operable to move inside the cylindrical groove **112,** based on the axial movement of the piston **108.** The sealing ring **110** is in contact with the piston **108.** Moreover, the sealing ring **110** is manufactured using rubber composition comprising hydrogenated nitrile butadiene rubber (HNBR), and carbon black.

It may be understood by a person skilled in the art that the FIG. 1 depicts the simplified illustration of the micropump **100** for delivery of the fluid for the sake of clarity only, which should not unduly limit the scope of the claims herein. The person skilled in the art will recognize many variations, alternatives, and modifications of embodiments of the present disclosure.

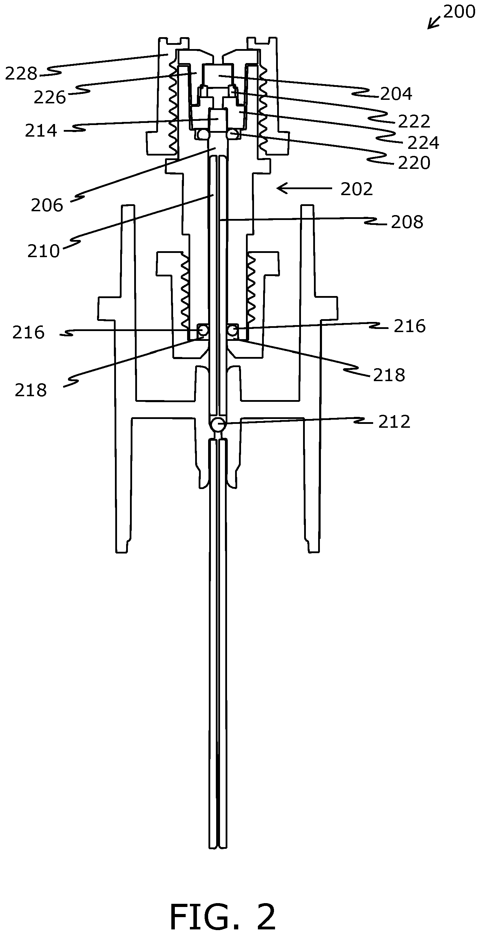

Referring to FIG. 2, there is shown a cross section of a micropump **200** in operation, for delivery of a fluid, in accordance with an exemplary implementation of the present disclosure. The micropump **200** comprises a body **202** comprising a metering chamber **206** for receiving the fluid, and a groove **218.** Typically, the fluid is delivered through a nozzle **204** attached downstream of the metering chamber **206.** The micropump **200** comprises a capillary **208** for delivering the fluid into the metering chamber **206.** The micropump **200** comprises a piston **210** operable to move axially inside the metering chamber **206.** The piston **210** has a piston face at a first end of the piston **210.** Specifically, an axial movement of the piston face along a path between a second end and a first end of the metering chamber **206** delivers the fluid through the nozzle **204.** As shown, the capillary **208** is implemented as a capillary bore **208** through the piston **210.** The capillary **208** comprises a valve arrangement **212** to restrict backward flow of the fluid from the metering chamber **206.** The micropump **200** further comprises a filter **214** arranged between the metering chamber **206** and the nozzle **204.** Furthermore, the groove **218** radially surrounds the piston **210** and has a common axis with the piston **210.** Specifically, the groove **218** houses a sealing ring **216,** which is an 'O' ring. The sealing ring **216** is operable to move inside the groove **218,** based on the axial movement of the piston **210,** wherein the sealing ring **216** is in contact with the piston **210,** against which it provides a sliding seal.

Another seal, outlet seal **220** may be provided between the body **202** and the chip holder **224,** near the outlet of the metering chamber **206.** The opposing surfaces of the outlet seal **220** form face seals with the body **202** and the chip holder **224.** Although this is a static seal, it may be constructed identically to the sealing ring **216,** both in terms of material and dimensions. Thus, the outlet seal is typically an 'O' ring.

Additionally, optionally, the micropump **200** comprises a gasket seal **222.** The opposing surfaces of the gasket seal **222** form face seals with the chip holder **224,** a nozzle holder **226** and the nozzle **204.** The gasket seal **224** is manufactured using a rubber composition the same as that used for the sealing ring **216,** and optionally the outlet seal **220.**

As shown in the exemplary implementation, the axial movement of the piston face of the piston **210** along the path between the second end and the first end of the metering chamber **206** causes an upward movement of the sealing ring **216** inside the groove **218.** It will be appreciated that an axial movement of the piston face of the piston **210** along a path between the first end and the second end of the metering chamber **206** causes a downward movement of the sealing ring **216** inside the groove **218.**

The body of the micropump shown in FIG. 2 also includes the nozzle holder **226.** Upper end-cap **228** has a screw thread by which it is engaged with the main component of the body **202.** The upper end-cap serves to retain the other components of the upper end of the body via the nozzle holder **226.**

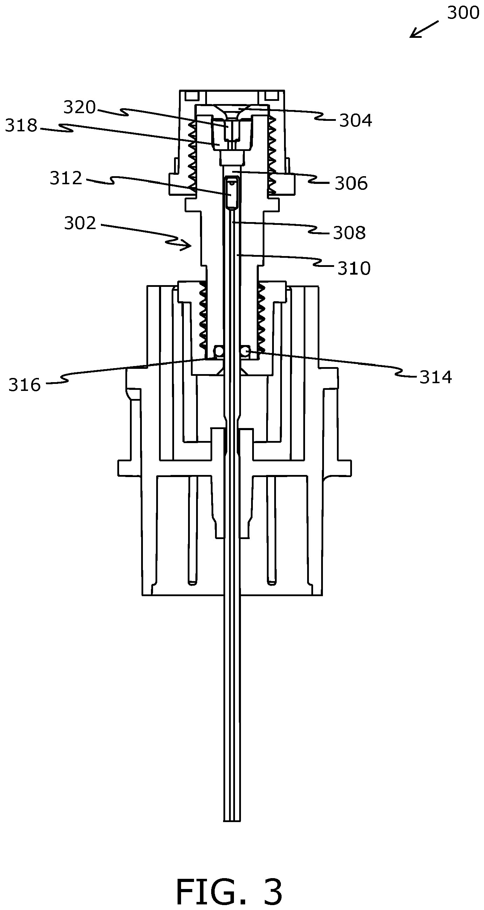

Referring to FIG. 3, there is shown a cross section of a micropump **300** in operation, for delivery of a fluid, in accordance with another exemplary implementation of the present disclosure. The micropump **300** comprises a body **302** comprising a metering chamber **306** for receiving the fluid, and a groove **316.** Typically, the fluid is delivered through a nozzle **304** attached downstream of the metering chamber **306.** The micropump **300** comprises a piston **310** operable to move axially inside the metering chamber **306.** The piston **310** has a piston face at a first end thereof. The micropump **300** comprises a capillary **308,** implemented as a capillary bore through the piston **310,** for delivering the fluid into the metering chamber **306.** Notably, an axial movement of the piston face along a path between a second end and a first end of the metering chamber **306** delivers the fluid through the nozzle **304.** The capillary **308** comprises a valve arrangement **312,** implemented at a proximal end of the capillary, to restrict backward flow of the fluid from the metering chamber **306.** Furthermore, the groove **316** radially surrounds the piston **310** and has a common axis with the piston **310.** Specifically, the groove **316** houses a sealing ring **314,** which is an 'O' ring. The sealing ring **314** is operable to move inside the groove **316,** based on the axial movement of the piston **310,** wherein the sealing ring **314** is in contact with the piston **310,** against which it provides a sliding seal. Additionally, the micropump **300** comprises a chip seal **318** providing an axial seal between the metering chamber and the nozzle. The chip seal **318** surrounds the chip **320** to create an annular radial seal.

It may be understood by a person skilled in the art that the FIGs. 2 and 3 depict simplified illustrations of the exemplary implementation of the micropump for delivery of the fluid for the sake of clarity only.

### EXAM PLE

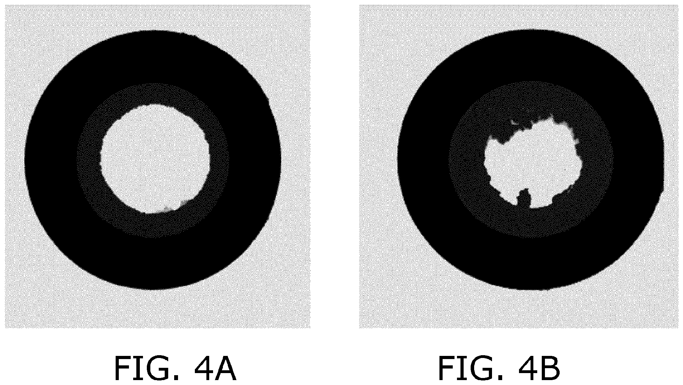

A micropump as described and illustrated in FIGs. 2 and 3 was tested by pumping for 100 actuations of fluid. The sealing ring was made of HNBR elastomer with a Shore A hardness of 80. FIG. 4A shows the condition of the seal after removing from the micropump and photographing under low magnification. A micropump of the prior art containing a sealing ring made of silicone rubber with a Shore A hardness of 55 was tested in the same way. FIG. 4B shows the condition of the seal after removing from the micropump and photographing under low magnification. It is evident that the sealing ring in FIG. 4A shows much less seal shredding of the internal bore than the sealing ring in FIG. 4B.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

### APPENDIX

A data analysis performed for a rubber composition in accordance with ASTM (American Society for Testing and Materials) Standard Test Methods is provided below. Furthermore, various data for the rubber composition have been recorded (stated herein below) based on tests and trials carried thereupon.

| Data sheet for Rubber Composition in 80 Durometer | | | |
|---|---|---|---|
| *Vulcanisation Conditions 170°C × 20 min* | | | |
| **Properties** | **Test Standard** | **Units** | **Value** |
| Tensile Strength | ASTM D412 DIN 53504 | MPa | 22.1 |
| Tear Strength | ASTM D624 Die B ISO 34 | kN/m | 7 |
| Modulus 100% | ASTM D412 DIN 53504 | MPa | 2.1 |
| Modulus 200% | ASTM D412 DIN 53504 | MPa | 5.8 |
| Modulus 300% | ASTM D412 DIN 53504 | MPa | 10.2 |
| Elongation at break | ASTM D412 DIN 53504 | % | 300 |
| Hardness | ASTM D2240 DIN 53505 | Shore A | 80 (Desired Value 80±5) |
| Compression Set (Method B, 70h at 150°C) | ASTM D395 DIN ISO 815 | % | 21 |
| Temperature | ASTM D 2137 | °C | -52°C to +160°C |

| *Resistance against Hot air (ASTM D 865 72 h at 150°C)* | | | |
|---|---|---|---|
| Change in Tensile Strength | DIN 53504 | % | +4 |
| Change in elongation at break | DIN 53504 | % | -6 |
| Hardness Change | DIN 53504 | Shore A | -9 |

| *Oil Resistance (ASTM D 471) in ASTM Oil No. 2, 7 days at 150°C* | | | |
|---|---|---|---|
| Change in Tensile Strength | DIN 53504 | % | +11 |
| Change in elongation at break | DIN 53504 | % | +16 |
| Hardness Change | DIN 53505 | Shore A | -9 |
| Volume Change | DIN ISO 1817 | % | +12 |

| *Oil Resistance (ASTM D 471) in ASTM Oil No. 3, 7 days at 150°C* | | | |
|---|---|---|---|
| Change in Tensile Strength | DIN 53504 | % | +11 |
| Change in elongation at break | DIN 53504 | % | +18 |
| Hardness Change | DIN 53505 | Shore A | -13 |
| Volume Change | DIN ISO 1817 | % | +25 |

## Claims

1. A micropump (200) for delivery of a fluid, comprising:
- a body (202) comprising a metering chamber (206) for receiving the fluid and a groove (218);
- a capillary (208) for delivering the fluid into the metering chamber (206);
- a nozzle (204) attached downstream of the metering chamber (206) for delivering the fluid from the metering chamber (206) therethrough;
- a piston (210) operable to move axially inside the metering chamber (206) and having a piston face at a first end of the piston (210), wherein an axial movement of the piston face along a path between a second end and a first end of the metering chamber (206) delivers the fluid through the nozzle (204);
wherein the groove (218) radially surrounds the piston (210) and has a common axis with the piston (210), wherein the groove (218) comprises a sealing ring (216), wherein the axial size of the groove (218) is greater than the axial size of the sealing ring (216) so that the sealing ring (216) is operable to move axially inside the groove (218) based on the axial movement of the piston (210), and wherein the sealing ring (216) is in contact with the piston (210) and the body (202);
wherein the sealing ring (216) comprises a rubber composition comprising:
- hydrogenated nitrile butadiene rubber (HNBR), and
- carbon black.

2. A micropump (200) of claim 1, wherein the capillary (208) is implemented as a capillary bore through the piston (210).

3. A micropump (200) of claim 1 or claim 2, wherein the capillary (208) comprises a valve arrangement (212) to restrict backward flow of the fluid from the metering chamber (206).

4. A micropump (200) of claim 1, 2 or 3, wherein the groove (218) is cylindrical.

5. A micropump (200) of any one of the preceding claims, wherein the concentration of the carbon black in the rubber composition is in a range of 40 to 60 parts per hundred parts of rubber (PHR) with respect to hydrogenated nitrile butadiene rubber (HNBR).

6. A micropump (200) of any one of the preceding claims, wherein the sealing ring (216) comprises a rubber composition comprising at least one metal oxide selected from zinc oxide, magnesium oxide.

7. A micropump (200) of claim 6, wherein concentration of the zinc oxide in the rubber composition is in a range of 1 to 3 parts per hundred parts of rubber (PHR) with respect to hydrogenated nitrile butadiene rubber (HNBR).

8. A micropump (200) of claim 6 or claim 7, wherein concentration of the magnesium oxide in the rubber composition is in a range of 1 to 3 parts per hundred parts of rubber (PHR) with respect to hydrogenated nitrile butadiene rubber (HNBR).

9. A micropump (200) of any one of the preceding claims, wherein the rubber composition comprises a rubber composition comprising a peroxide crosslinking agent.

10. A micropump (200) of claim 9, wherein the peroxide cross linking agent is bis(t-butylperoxy isopropyl) benzene (BIPB), and the concentration of the cross linking agent in the rubber composition is in a range of 2 to 4 parts per hundred parts of rubber (PHR) with respect to hydrogenated nitrile butadiene rubber (HNBR).

11. A micropump (200) of any one of the preceding claims, wherein the rubber composition of the sealing ring (216) comprises a rubber composition comprising at least one of: anti-ager dialkylated diphenylamine (DDA), anti-ager zinc di(benzimidazole-2-yl)disulphide (MBZ), cross-linking agent Triallyl isocyanurate (TAIC).

12. A micropump (200) of any one of the preceding claims, wherein the fluid comprises a pharmaceutical compound.

13. A micropump (200) of any one of the preceding claims, wherein the sealing ring (216) provides at least one of: a radial seal, an axial seal with the piston (210).

14. A micropump (200) of any one of the preceding claims, wherein the body (202) includes two parts that are assembled to define the groove (218) and retain the sealing ring (216).

15. A micropump (200) of any one of the preceding claims, further comprising a gasket seal (222), wherein opposing surfaces of the gasket seal (222) form face seals with the body (202) and the nozzle (204), and wherein the gasket seal (222) comprises a rubber composition the same as that used for the sealing ring (216).

## Patentansprüche

1. Mikropumpe (200) zum Fördern eines Fluids, umfassend:
- einen Körper (202), der eine Dosierkammer (206) zum Aufnehmen des Fluids und eine Nut (218) umfasst;
- eine Kapillare (208) zum Fördern des Fluids in die Dosierkammer (206);
- eine Düse (204), die nach der Dosierkammer (206) angebracht ist, um das Fluid von der Dosierkammer (206) durch diese zu fördern;
- einen Kolben (210), der betreibbar ist, sodass er sich axial innerhalb der Dosierkammer (206) bewegt, und der eine Kolbenfläche an einem ersten Ende des Kolbens (210) aufweist, wobei eine axiale Bewegung der Kolbenfläche entlang eines Wegs zwischen einem zweiten Ende und einem ersten Ende der Dosierkammer (206) das Fluid durch die Düse (204) fördert;
wobei die Nut (218) den Kolben (210) radial umgibt und eine gemeinsame Achse mit dem Kolben (210) aufweist, wobei die Nut (218) einen Dichtring (216) umfasst, wobei die axiale Größe der Nut (218) größer ist als die axiale Größe des Dichtrings (216), sodass der Dichtring (216) derart betreibbar ist, dass er sich basierend auf der axialen Bewegung des Kolbens (210) axial innerhalb der Nut (218) bewegt, und wobei der Dichtring (216) in Kontakt mit dem Kolben (210) und dem Körper (202) steht;
wobei der Dichtring (216) eine Kautschukzusammensetzung umfasst, die umfasst:
- hydrierten Nitrilbutadienkautschuk (HNBR) und
- Ruß.

2. Mikropumpe (200) nach Anspruch 1, wobei die Kapillare (208) als eine Kapillarbohrung durch den Kolben (210) implementiert ist.

3. Mikropumpe (200) nach Anspruch 1 oder Anspruch 2, wobei die Kapillare (208) eine Ventilanordnung (212) umfasst, um eine Rückwärtsströmung des Fluids von der Messkammer (206) zu begrenzen.

4. Mikropumpe (200) nach Anspruch 1, 2 oder 3, wobei die Nut (218) zylindrisch ist.

5. Mikropumpe (200) nach einem der vorstehenden Ansprüche, wobei die Konzentration des Rußes in der Kautschukzusammensetzung in einem Bereich von 40 bis 60 Teilen pro hundert Teile Kautschuk (PHR) in Bezug auf hydrierten Nitrilbutadienkautschuk (HNBR) liegt.

6. Mikropumpe (200) nach einem der vorstehenden Ansprüche, wobei der Dichtungsring (216) eine Kautschukzusammensetzung umfasst, die mindestens ein Metalloxid umfasst, das aus Zinkoxid und Magnesiumoxid ausgewählt ist.

7. Mikropumpe (200) nach Anspruch 6, wobei die Konzentration des Zinkoxids in der Kautschukzusammensetzung in einem Bereich von 1 bis 3 Teilen pro hundert Teile Kautschuk (PHR) in Bezug auf hydrierten Nitrilbutadienkautschuk (HNBR) liegt.

8. Mikropumpe (200) nach Anspruch 6 oder Anspruch 7, wobei die Konzentration des Magnesiumoxids in der Kautschukzusammensetzung in einem Bereich von 1 bis 3 Teilen pro hundert Teile Kautschuk (PHR) in Bezug auf hydrierten Nitrilbutadienkautschuk (HNBR) liegt.

9. Mikropumpe (200) nach einem der vorstehenden Ansprüche, wobei die Kautschukzusammensetzung eine ein Peroxidvernetzungsmittel umfassende Kautschukzusammensetzung umfasst.

10. Mikropumpe (200) nach Anspruch 9, wobei das Peroxidvernetzungsmittel Bis(t-butylperoxyisopropyl)benzol (BIPB) ist und die Konzentration des Vernetzungsmittels in der Kautschukzusammensetzung in einem Bereich von 2 bis 4 Teilen pro hundert Teile Kautschuk (PHR) in Bezug auf hydrierten Nitrilbutadienkautschuk (HNBR) liegt.

11. Mikropumpe (200) nach einem der vorstehenden Ansprüche, wobei die Kautschukzusammensetzung des Dichtungsrings (216) eine Kautschukzusammensetzung umfasst, die mindestens eines umfasst von: Alterungsschutzmittel dialkyliertes Diphenylamin (DDA), Alterungsschutzmittel Zinkdi(benzimidazol-2-yl)disulfid (MBZ), Vernetzungsmittel Triallylisocyanurat (TAIC).

12. Mikropumpe (200) nach einem der vorstehenden Ansprüche, wobei das Fluid eine pharmazeutische Verbindung umfasst.

13. Mikropumpe (200) nach einem der vorstehenden Ansprüche, wobei der Dichtungsring (216) mindestens eines bereitstellt von: einer Radialdichtung, einer Axialdichtung mit dem Kolben (210).

14. Mikropumpe (200) nach einem der vorstehenden Ansprüche, wobei der Körper (202) zwei Teile umfasst, die zusammengefügt sind, um die Nut (218) zu definieren und den Dichtungsring (216) zurückzuhalten.

15. Mikropumpe (200) nach einem der vorstehenden Ansprüche, ferner umfassend eine Pressdichtung (222), wobei entgegengesetzte Flächen der Pressdichtung (222) Flächendichtungen mit dem Körper (202) und der Düse (204) bilden, und wobei die Pressdichtung (222) eine gleiche Kautschukzusammensetzung wie die für den Dichtungsring (216) verwendete umfasst.

## Revendications

1. Micropompe (200) pour la distribution d'un fluide, comprenant :
- un corps (202) comprenant une chambre de dosage (206) pour recevoir le fluide et une rainure (218) ;
- un capillaire (208) pour distribuer le fluide dans la chambre de dosage (206) ;
- une buse (204) fixée en aval de la chambre de dosage (206) pour distribuer le fluide depuis la chambre de dosage (206) à travers celle-ci ;
- un piston (210) pouvant fonctionner pour se déplacer axialement à l'intérieur de la chambre de dosage (206) et ayant une face de piston au niveau d'une première extrémité du piston (210), dans laquelle un mouvement axial de la face de piston le long d'un trajet entre une deuxième extrémité et une première extrémité de la chambre de dosage (206) distribue le fluide à travers la buse (204) ;
dans laquelle la rainure (218) entoure radialement le piston (210) et a un axe commun avec le piston (210), dans laquelle la rainure (218) comprend une bague d'étanchéité (216), dans laquelle la taille axiale de la rainure (218) est supérieure à la taille axiale de la bague d'étanchéité (216) de sorte que la bague d'étanchéité (216) peut fonctionner pour se déplacer axialement à l'intérieur de la rainure (218) sur la base du mouvement axial du piston (210), et dans laquelle la bague d'étanchéité (216) est en contact avec le piston (210) et le corps (202) ;
dans laquelle la bague d'étanchéité (216) comprend une composition de caoutchouc comprenant :
- du caoutchouc nitrile butadiène hydrogéné (HNBR), et
- du noir de carbone.

2. Micropompe (200) selon la revendication 1, dans laquelle le capillaire (208) est mis en œuvre sous la forme d'un alésage capillaire à travers le piston (210).

3. Micropompe (200) selon la revendication 1 ou la revendication 2, dans laquelle le capillaire (208) comprend un agencement de soupape (212) pour restreindre l'écoulement en retour du fluide depuis la chambre de dosage (206).

4. Micropompe (200) selon la revendication 1, 2 ou 3, dans laquelle la rainure (218) est cylindrique.

5. Micropompe (200) selon l'une quelconque des revendications précédentes, dans laquelle la concentration du noir de carbone dans la composition de caoutchouc est dans une plage de 40 à 60 parties pour cent parties de caoutchouc (PHR) par rapport au caoutchouc nitrile butadiène hydrogéné (HNBR).

6. Micropompe (200) selon l'une quelconque des revendications précédentes, dans laquelle la bague d'étanchéité (216) comprend une composition de caoutchouc comprenant au moins un oxyde métallique choisi parmi l'oxyde de zinc, l'oxyde de magnésium.

7. Micropompe (200) selon la revendication 6, dans laquelle la concentration de l'oxyde de zinc dans la composition de caoutchouc est dans une plage de 1 à 3 parties pour cent parties de caoutchouc (PHR) par rapport au caoutchouc nitrile butadiène hydrogéné (HNBR).

8. Micropompe (200) selon la revendication 6 ou la revendication 7, dans laquelle la concentration de l'oxyde de magnésium dans la composition de caoutchouc est dans une plage de 1 à 3 parties pour cent parties de caoutchouc (PHR) par rapport au caoutchouc nitrile butadiène hydrogéné (HNBR).

9. Micropompe (200) selon l'une quelconque des revendications précédentes, dans laquelle la composition de caoutchouc comprend une composition de caoutchouc comprenant un agent de réticulation de peroxyde.

10. Micropompe (200) selon la revendication 9, dans laquelle l'agent de réticulation de peroxyde est le bis(t-butylperoxy isopropyl) benzène (BIPB), et la concentration de l'agent de réticulation dans la composition de caoutchouc est dans une plage de 2 à 4 parties pour cent parties de caoutchouc (PHR) par rapport au caoutchouc nitrile butadiène hydrogéné (HNBR).

11. Micropompe (200) selon l'une quelconque des revendications précédentes, dans laquelle la composition de caoutchouc de la bague d'étanchéité (216) comprend une composition de caoutchouc comprenant au moins l'un parmi : antioxydant diphénylamine dialkylée (DDA), antioxydant di(benzimidazole-2-yl)disulfure de zinc (MBZ), agent de réticulation Triallyle isocyanurate (TAIC).

12. Micropompe (200) selon l'une quelconque des revendications précédentes, dans laquelle le fluide comprend un composé pharmaceutique.

13. Micropompe (200) selon l'une quelconque des revendications précédentes, dans laquelle la bague d'étanchéité (216) fournit au moins l'un parmi : un joint radial, un joint axial avec le piston (210) .

14. Micropompe (200) selon l'une quelconque des revendications précédentes, dans laquelle le corps (202) comporte deux parties qui sont assemblées pour définir la rainure (218) et retenir la bague d'étanchéité (216).

15. Micropompe (200) selon l'une quelconque des revendications précédentes, comprenant en outre un joint d'étanchéité (222), dans laquelle des surfaces opposées du joint d'étanchéité (222) forment des joints d'étanchéité de face avec le corps (202) et la buse (204), et dans laquelle le joint d'étanchéité (222) comprend une composition de caoutchouc identique à celle utilisée pour la bague d'étanchéité (216).
